# EUROPEAN PATENT APPLICATION

(11) **EP 4 382 076 A1**
(43) Date of publication of application: **12.06.2024**
(21) Application number: 23214524.3
(22) Date of filing: 06.12.2023
(51) Int. Cl.: A61C 19/045, A61B 5/11, A61B 5/00

(54) **APPARATUS AND METHOD FOR TRACKING JAW MOTION AND SENSOR ASSEMBLY THEREFOR**

(30) Priority: 07.12.2022 KR 20220169440; 31.03.2023 KR 20230042498
(71) Applicant: Dentium Co., Ltd., Gyeonggi-do 16229 (KR)
(72) Inventor: Lee, In Jae, 16229 Suwon-si,Gyeonggi-do (KR); Sung, Mun Hyun, 16229 Suwon-si,Gyeonggi-do (KR); Choi, Young Hee, 16507 Suwon-si, Gyeonggi-do (KR); Heo, Jun Mu, 17056 Yongin-si, Gyeonggi-do (KR); Kim, Min Ji, 16509 Suwon-si, Gyeonggi-do (KR); Jung, Won Jae, 16705 Suwon-si,Gyeonggi-do, (KR); Kim, Kyung Hoon, 16636 Suwon-si, Gyeonggi-do (KR); Kang, Hyeon Woo, 16237 Suwon-si,Gyeonggi-do (KR)
(74) Representative: von Bülow & Tamada

(57) **Abstract**

An apparatus for tracking jaw motion according to the present invention includes: a base that is configured to generate an electromagnetic field; a first sensor unit that is attached to a maxillary tooth of a patient and includes a first sensor detecting the electromagnetic field generated from the base; a second sensor unit that is attached to a mandibular tooth of the patient and includes a second sensor detecting the electromagnetic field generated from the base; and a control unit that is configured to receive a detection signal from the first sensor and a detection signal from the second sensor, respectively, and calculate and provide a result of tracking a motion of a mandible with respect to a maxilla.

## Description

### 1. FIELD

The present invention relates to an apparatus and method for tracking jaw motion, and a sensor assembly therefor.

### 2. DESCRIPTION OF RELATED ART

Teeth occlusion refers to engagement of maxilla and mandible, and it is important to achieve accurate occlusion when correcting teeth or performing prosthetics. Since the motion of the mandible is a complex result of actions of a temporomandibular joint (TMJ) and masticatory muscles controlled by an occlusal nerve, the aspect of the motion of the mandible is complex. The motion of the mandible may be depicted three-dimensionally on a motion plane, and may be divided into rotation movement in which a condyle moves around a limited axis, and translation movement in which the condyle is shifted.

The temporomandibular joint (TMJ) serves to allow the lower jaw (mandible) to move, and is the most frequently moved joint in the middle of a body. The temporomandibular joint is also called a "ball and socket" joint, and a rounded end or "ball" part of the temporomandibular joint is called a mandibular condyle and the socket part thereof is called an articular fossa. There is an articular disc composed of cartilage between the mandibular condyle and the articular fossa, and the articular disc acts as a cushion to absorb stress. In addition, the articular disc helps the mandibular condyle move easily when opening or closing a mouth.

The teeth occlusion through the movement of the temporomandibular joint may be diagnosed or identified in several ways. Such example is a dental articulator, which is a machine that reproduces/imitates the mandibular movement by fixing maxillary and mandibular teeth models in a three-dimensional positional relationship like a human body. The dental articulator is used for the purpose of maintaining a cast (correction) of the maxilla and mandible in a condition of a patient, diagnosing an occlusal status of natural teeth and artificial teeth while simulating the jaw movement, or making the correction based on a dentition relationship between the natural teeth and artificial teeth, or the like.

In humans, the maxilla and mandible are not separated from each other, but are connected through the temporomandibular joint of the skull. Some patients have a floating temporomandibular joint, which causes front teeth to spread as the temporomandibular joint moves to a stable position.

To prevent the dentition from being disturbed, an orthodontic doctor should be able to predict and evaluate the change in the temporomandibular joint and the movement above the center before orthodontic treatment. During the orthodontic diagnosis, the dental articulator is used to confirm the position of the temporomandibular joint.

In order to identify the diagnosis process and post-treatment results of temporomandibular joint disorders, the range of the mandible movement is examined using a dental articulator. As a method of examining a range of mandible movement, a method of dynamically visualizing 3D model displacement using optical scanning and combining results of graphical analysis of feature points such as interincisal points or dondyle, a method of measuring jaw movement using ultrasound, or the like is presented. However, there is a disadvantage in that, in the former case, the use of markers increases to affect or overcome blind spots, and in the latter case, a complicated preparation process is required, and a huge device worn by a patient also hinders convenience.

### SUMMARY

The present invention provides an apparatus and method for tracking jaw motion capable of simply configuring the apparatus and accurately analyzing natural movement of mandible without the influence of blind spots by forming a sensor, which detects an electromagnetic field, in the form of a small sensor unit attached to maxillary and mandibular teeth.

The present invention is to increase usability of an apparatus by generating a crown from results of deriving an exact position and shape of an occlusal surface when maxilla and mandible are occluded.

The present invention provides a sensor assembly capable of minimizing discomfort caused by a cable connecting a sensor while accurately detecting movement of a jaw of a patient. The present invention is to simply configure the apparatus and accurately analyze the natural movement of the mandible without the influence of the blind spots by attaching the sensor, which detects the electromagnetic field, to the maxillary and mandibulary teeth in the form of a small tray using the sensor assembly.

The problems of the present invention are not limited to those mentioned above, and other technical problems will be clearly understood by those skilled in the art from the following description.

According to an aspect of the present invention, an apparatus for tracking jaw motion includes: a base that is configured to generate an electromagnetic field; a first sensor unit that is attached to a maxillary tooth of a patient and includes a first sensor detecting the electromagnetic field generated from the base; a second sensor unit that is attached to a mandibular tooth of the patient and includes a second sensor detecting the electromagnetic field generated from the base; and a control unit that is configured to receive a detection signal from the first sensor and a detection signal from the second sensor, respectively, and calculate and provide a result of tracking a motion of a mandible with respect to a maxilla.

The first sensor unit may include: a first fixing part that is attached to the maxillary tooth; and a first sensor part that is detachably configured to the first fixing part, and the second sensor unit may include: a second fixing part that is attached to the mandibular tooth; and a second sensor part that is detachably configured to the second fixing part.

The first fixing part may include a first adhesive surface for attachment to the maxillary tooth by an orthodontic adhesive, and the second fixing part may include a second adhesive surface for attachment to the mandibular tooth by the orthodontic adhesive.

The first sensor part may include a first slide groove that is configured to be inserted into the first fixing part in a slide manner, and the second sensor part may include a second slide groove that is configured to be inserted into the second fixing part in the slide manner.

A first protruding part protruding toward a first slide groove may be formed on the first fixing part, and a second protruding part protruding toward a second slide groove may be formed on the second fixing part.

A first through hole may be formed at a rear of the first protruding part, and a second through hole may be formed at a rear of the second protruding part.

A pair of first openings communicating with the first through hole may be formed on both sides of the first protrusion, and a pair of second openings communicating with the second through hole may be formed on both sides of the second protrusion.

A first locking groove in which the first protrusion is caught in a snap fit shape may be formed in the first slide groove, and a second locking groove in which the second protrusion is caught in a snap fit shape may be formed in the second slide groove.

A plurality of first magnetic beads may be arranged to be spaced apart from each other in the first sensor part, and a plurality of second magnetic beads may be arranged to be spaced apart from each other in the second sensor part.

A plurality of third magnetic beads may be arranged to be spaced apart from each other in the first fixing part, and a plurality of fourth magnetic beads may be arranged to be spaced apart from each other in the second fixing part. The plurality of third beads and the plurality of fourth beads may be configured to be captured by an imaging device, and the control unit may control to generate a reference position from the plurality of third beads and the plurality of fourth beads captured by the imaging device.

When the first sensor part is inserted into the first fixing part, the plurality of third beads may be formed so that positions of the plurality of third beads on a front view overlap with the plurality of first beads, and when the second sensor part is inserted into the second fixing part, the plurality of fourth beads may be formed so that positions of the plurality of fourth beads on a front view overlap with the plurality of second beads.

Each body constituting the first fixing part and the second fixing part may be made of a non-magnetic material, and each housing supporting components included in the first sensor part and the second sensor part may be made of the non-magnetic material.

According to another aspect of the present invention, a method for tracking jaw motion includes: arranging a base that is configured to generate an electromagnetic field; attaching a first sensor detecting the electromagnetic field generated from the base to a maxillary tooth of a patient; attaching a second sensor detecting the electromagnetic field generated from the base to a mandibular tooth of the patient; and receiving a detection signal from the first sensor and a detection signal from the second sensor, respectively, and calculating and providing a result of tracking a motion of a mandible with respect to a maxilla.

The method may further include automatically generating a crown from a result of deriving a position and shape of an occlusal surface when the maxilla and the mandible are occluded.

According to still another aspect of the present invention, a sensor assembly for tracking jaw motion includes: a first sensor unit that is attached to a maxillary tooth of a patient and configured to detect an electromagnetic field generated from a base; a first cable that transmits a detection signal of the first sensor unit; a second sensor unit that is attached to a mandibular tooth of the patient and configured to detect the electromagnetic field generated from the base; a second cable that transmits a detection signal of the second sensor unit; a neckband that is configured to be worn around a neck of the patient and carries the first cable and the second cable; and an interface unit that is provided at a portion of the neckband and configured to allow the cables to be connected to an external processing device.

The neckband may be formed in a 'C' shape that is supported by being wrapped around a back of the neck of the patient, and may have a front portion provided with a first end portion to which the first cable is connected and a second end portion to which the second cable is connected.

The neckband may be formed so that the first cable and the second cable follow the neckband from the first end portion and the second end portion provided at the front portion thereof, respectively, or are each arranged inside the neckband.

The first sensor unit and the second sensor unit may each be configured to be detachably coupled to a fixing part attached to teeth of the patient.

### BRIEF DESCRIPTION OF DRAWINGS

FIG. 1 is a conceptual diagram illustrating an apparatus for tracking jaw motion according to an example of the present invention.
FIG. 2 is a front view illustrating a state in which each fixing part constituting a sensor unit related to the present invention is exemplarily attached to maxillary and mandibular teeth, respectively.
FIGS. 3A and 3B are front views exemplarily illustrating a state in which a sensor part is mounted on each fixing part in the state of FIG. 2.
FIGS. 4A and 4B are side cross-sectional views corresponding to FIGS. 3A and 3B, respectively.
FIG. 5 is a perspective view conceptually illustrating the attachment of the fixing part constituting the sensor unit related to the present invention to tooth.
FIG. 6 is a perspective view illustrating a state in which the fixing part and the sensor part are separated to describe mounting the sensor part on the fixing part.
FIG. 7 is an example related to the present invention, and is a perspective view illustrating the sate in which the sensor part and the fixing part are separated to show the arrangement of a plurality of beads included in the sensor part and a plurality of beads included in the fixing part.
FIG. 8 is a perspective view of a sensor assembly for tracking jaw motion according to an embodiment of the present invention.
FIG. 9 is a diagram illustrating an appearance in which a first sensor unit and a second sensor unit are attached to maxillary and mandibular teeth, respectively, while a patient is wearing the sensor assembly of FIG. 8.
FIG. 10 is a front view illustrating an exemplary state in which the first sensor unit and the second sensor unit in FIG. 9 are attached to the maxillary and mandibular teeth, respectively.
FIG. 11 is a diagram illustrating the state in which the fixing part and the sensor unit are separated to describe mounting the sensor unit on the fixing part in FIG. 9.
FIG. 12 is a perspective view illustrating the state in which the sensor unit and the fixing part are separated to show the arrangement of the plurality of beads included in the sensor unit and the plurality of beads included in the fixing part.

### DETAILED DESCRIPTION

Hereinafter, an apparatus and method for tracking jaw motion related to the present invention and a sensor assembly therefor will be described in detail with reference to the attached drawings. Terms used in the specification and claims may not be limited to dictionary meanings, and may be appropriately defined and understood to explain the present invention in the best way.

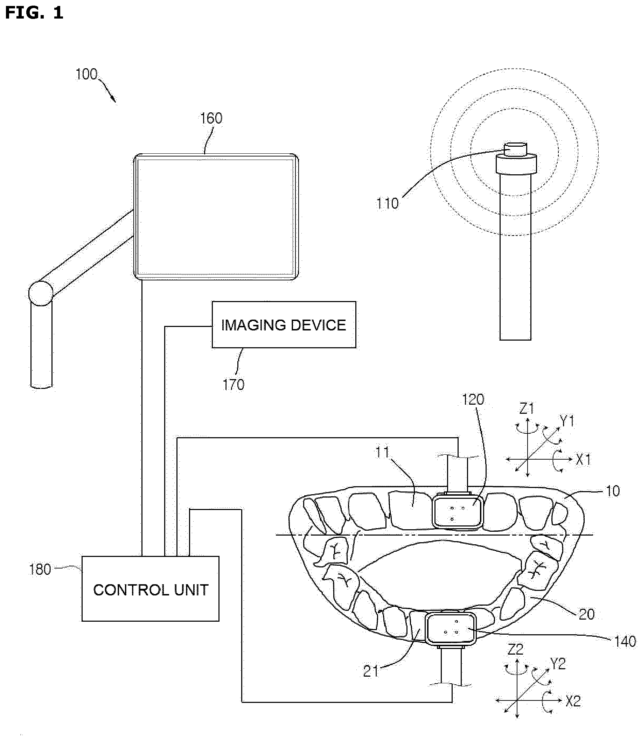

FIG. 1 conceptually illustrates an apparatus 100 for tracking jaw motion related to the present invention. As illustrated in FIG. 1, the apparatus 100 for tracking jaw motion includes a base 110 configured to generate an electromagnetic field. The base 110 is configured to generate a magnetic field or electromagnetic field (EM). The base 110 may be installed at a certain height (e.g., 750 mm) on one side of a chair or bed where a patient is located, which will be described later.

In order to measure the motion of a maxilla 10 and mandible 20 of a patient in real time, a first sensor unit 120 and a second sensor unit 140 are installed on a tooth 11 of the maxilla 10 and a tooth 21 of the mandible 20, respectively.

The first sensor unit 120 and the second sensor unit 140 may each include a magnetic sensor or an electromagnetic sensor for detecting the magnetic field or electromagnetic field of the base 110. The first sensor unit 120 detects the movement or change in posture (roll, pitch, yaw, etc.) of the maxilla 10 independent of the second sensor unit 140 (based on a coordinate system defined by X1, Y1, and Z1 axes), and the second sensor unit 140 detects the movement or change in posture of the mandible 20 independent of the first sensor unit 120 (based on a coordinate system defined by X2, Y2, and Z2 axes).

The first sensor unit 120 and the second sensor unit 140 have a structure that are attached to the teeth 11 and 21 of the maxilla 10 and the mandible 20, and may be similar in size to the teeth 11 and 21 as a whole or may have a relatively small size within 2 to 3 times the size of the teeth 11 and 21.

The first sensor unit 120 and the second sensor unit 140 are connected to a control unit 180, and the control unit 180 may be configured to signals detected by the first sensor unit 120 and the second sensor unit 140, respectively, calculate these signals to determine a direction and distance of the movement of the mandible 20 with respect to the maxilla 10 and a position and shape of an occlusal surface in real time, and visually output the determined direction and distance and position and shape through a display 160, etc.

The apparatus 100 for tracking jaw motion in this example may include an imaging device 170 for capturing parts to which the sensor units 120 and 140 are attached to check reference positions of the maxilla 10 and mandible 20 of the patient or an occlusal state of the maxilla 10 and mandible 20. The imaging device 170 may be a variety of devices for obtaining images, including a computed tomography device, an intraoral scanner, or the like.

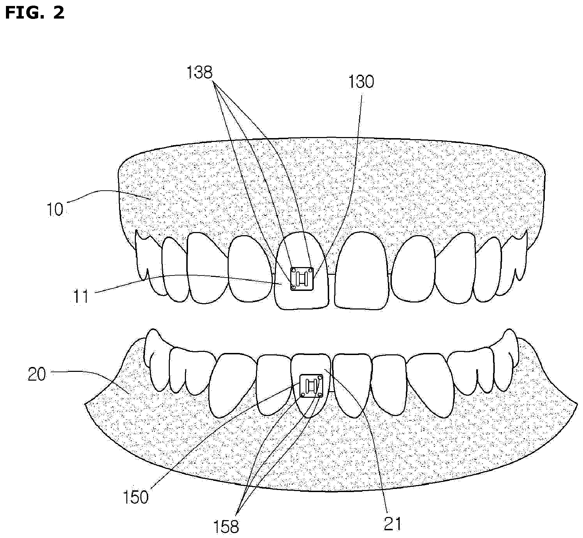

FIGS. 2 to 4 are diagrams illustrating that a first sensor part 121 and a first fixing part 130 constituting the first sensor unit 120 related to the present invention and a second sensor part 141 and a second fixing part 150 constituting the second sensor unit 140 are attached to the tooth 11 of the maxilla 10 and the tooth 21 of the mandible 20, respectively, and a patient moves a jaw.

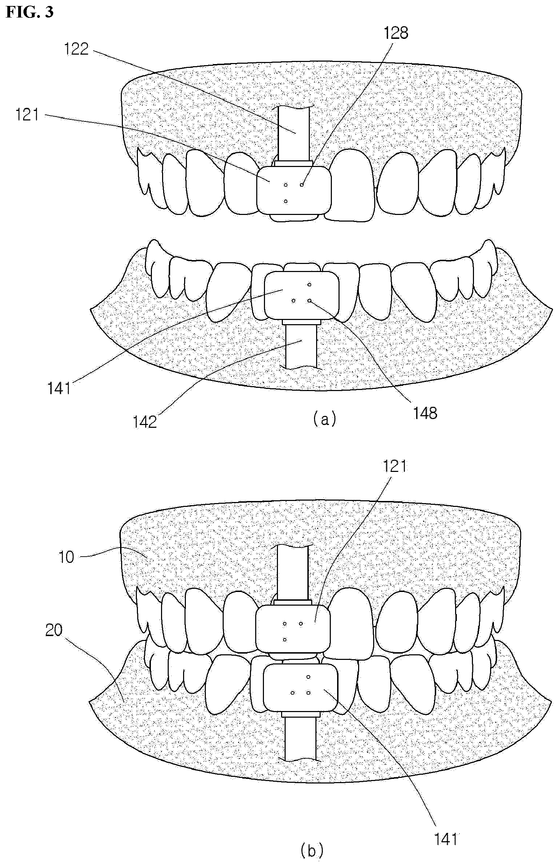

As illustrated in FIG. 2, the first fixing part 130 included in the first sensor unit 120 (FIG. 1) and the second fixing part 150 included in the second sensor unit 140 (FIG. 1) are formed in a relatively small size compared to the teeth 11 and 21 to which the first fixing part 130 and the second fixing part 150 will be attached. The fixing parts 130 and 150 may be attached to anterior teeth, incisors, or healthy teeth of the maxilla 10 and mandible 20, and in some cases, may also be attached to molars. The fixing parts 130 and 150 are formed so that the fixing parts 130 and 150 may be attached to either a front surface or a side surface of the teeth 11 and 21 without surrounding the teeth 11 and 21, and then has a structure that makes it easy to fasten small sensor parts 121 and 141 (FIG. 3B). As illustrated in FIG. 2, a plurality of beads 138 and 158 are arranged to be spaced apart from each other in the first fixing part 130 and the second fixing part 150, respectively. These beads 138 and 158 are formed in the form of a magnetic metal bead. Measurement data may be matched using only the beads 138 and 158 of the fixing parts 130 and 150 without the plurality of beads 138 and 158 fastening the sensor parts 121 and 141. That is, the plurality of beads 138 and 158 may be captured by the imaging device 170 (FIG. 1) described above, and the control unit 180 controls to generate the reference position from the plurality of beads 138 and 158 captured by the imaging device 170.

As illustrated in FIGS. 3A and 3B, the first sensor part 121 having a first cable 122 and the second sensor part 141 having a second cable 142 are mounted on the first fixing part 130 and the second fixing part 150, respectively, while the first fixing part 130 and the second fixing part 150 are attached to the teeth 11 and 21, respectively. A plurality of beads 128 and 148 may be arranged to be spaced apart from each other in the first sensor part 121 and the second sensor part 141 to match the measurement data. These beads 128 and 148 may be formed in the form of the magnetic metal bead and may have a triangular arrangement with a certain height difference in relation to each sensor inside. In this case, each body constituting the first fixing part 130 and the second fixing part 150 may be made of a non-magnetic material (e.g., titanium, synthetic resin, ceramic, etc.) to minimize magnetic interference, and each housing supporting parts included in the first sensor part 121 and the second sensor part 141 may also be made of non-magnetic material.

By doing this, when a patient moves the maxilla 10 and mandible 20 while the first sensor part 121 and the second sensor part 141 are attached to the maxilla 10 and mandible 20, respectively, measured values of the first sensor part 121 and the second sensor part 141 are transmitted to the control unit 180, so the exact position, direction, and movement distance of the maxilla 10 and mandible 20 are derived in real time.

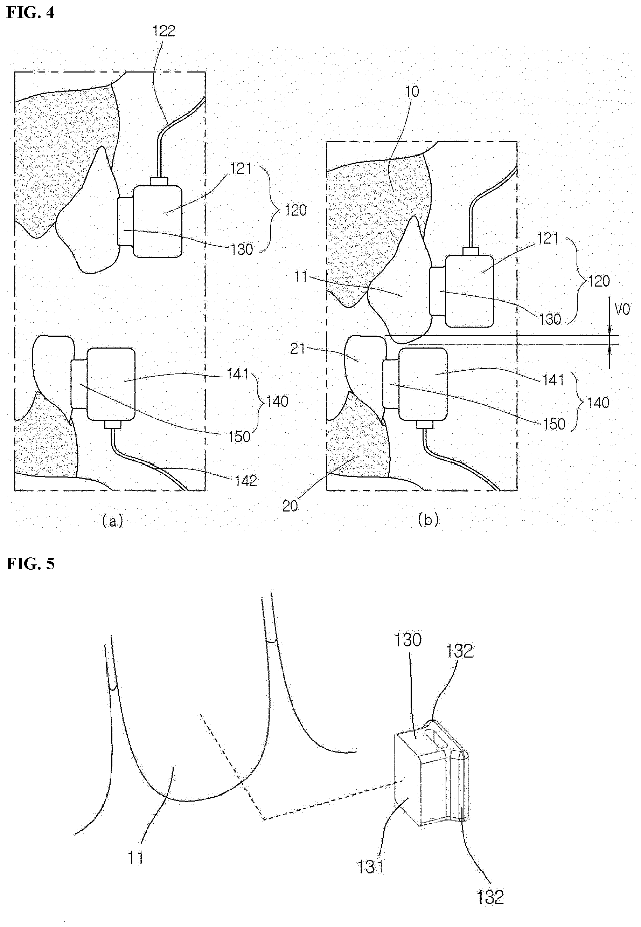

FIGS. 4A and 4B are side cross-sectional views corresponding to FIGS. 3A and 3B, respectively.

As illustrated in FIG. 4A, as a patient opens his/her mouth, the first sensor part 121 and the second sensor part 141 attached to the maxilla 10 and mandible 20, respectively, move together, and the first sensor part 121 and the second sensor part 141 transmit sensing data to the control unit 180 to identify the change in the relative position between the maxilla 10 and mandible 20 in real time.

As illustrated in FIG. 4B, when the maxilla 10 and the mandible 20 are occluded with each other, the first sensor unit 120 and the second sensor unit 140 may have a spatial distance to the extent that the tooth 11 of the maxilla 10 and the tooth 21 of the mandible 20 do not interfere with each other due to a naturally overlapping width VO.

FIG. 5 illustrates attaching the first fixing part 130 to the tooth 11. The first fixing part 130 has an adhesive surface 131 for being attached to a surface of the tooth 11 by an orthodontic adhesive. Slide protrusions 132 into which the first sensor part 121 is inserted may be formed on both left and right sides of the other side of the adhesive surface 131.

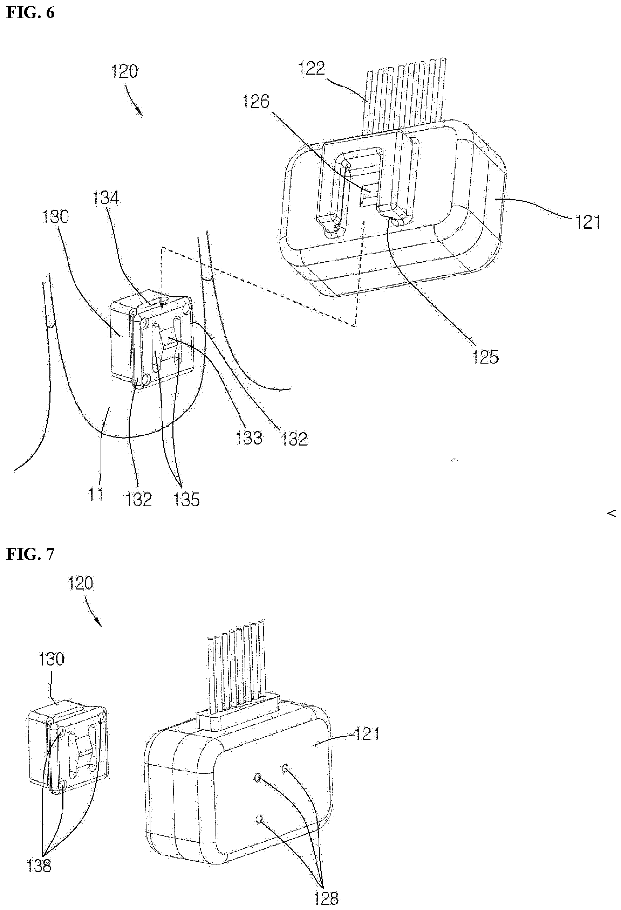

As illustrated in FIG. 6, the first sensor part 121 is slid and fastened from above while the first fixing part 130 is attached to the tooth 11 by the orthodontic adhesive. The fixing method of the first sensor part 121 may also be called a dovetail method. A slide groove 125 is formed in the first sensor part 121 in response to the slide protrusion 132 of the first fixing part 130.

In order to maintain the state where the first sensor part 121 is inserted into the first fixing part 130, the first fixing part 130 is formed with a protruding part 133 that protrudes toward the slide groove 125. A locking groove 126 is formed in the slide groove 125 so that the protruding part 133 is caught in a snap fit or dovetail form. A through hole 134 may be formed at a rear of the protruding part 133 so that the protruding part 133 can be elastically deformed and supported in this process, and through holes 134 and a pair of openings 135 communicating with the through hole 134 may be formed on both sides of the protruding part 133. The protruding part 133, the through hole 134, and the pair of openings 135 form a structure that easily fastens the first sensor part 121 to the first fixing part 130 in the snap fit form. The slide groove 125 may be formed with a closed upper end so as to limit a final position when the first sensor part 121 is pushed into the first fixing part 130.

As illustrated in FIG. 7, the plurality of beads 128 described above are arranged on the surface of the first sensor part 121. Separately from this, the plurality of beads 138 having the same arrangement as the beads 128 of the first sensor part 121 are arranged in the first fixing part 130. The plurality of beads 138 of the first fixing part 130 are formed in the form of the magnetic metal bead so that they may be captured by the imaging device 170 (FIG. 1). The imaging device 170 refers to a computed tomography (CT) device, a scanner for intraoral imaging (IOS), or the like. The control unit 180 (FIG. 1) controls to generate the reference position from the plurality of beads 138 of the first fixing part 130 captured by the imaging device 170. In this case, when the first sensor part 121 is inserted into the first fixing part 130, the plurality of beads 138 of the first fixing part 130 are arranged so that the positions of the plurality of beads 138 of the first fixing part 130 in the front view overlap with the plurality of beads 128 of the first sensor part 121.

Similar to the first sensor part 121 and the first fixing part 130 illustrated in FIGS. 5 to 7, the second sensor part 141 and the second fixing part 150 also include components corresponding to the first sensor part 121 and the first fixing part 130. This is replaced with the descriptions of the first sensor part 121 and the first fixing part 130.

In this way, the first sensor unit 120 and the second sensor unit 140, which detect the magnetic field or electromagnetic field of the base 110, are attached to the tooth 11 of the maxilla 10 and the tooth 21 of the mandible 20, respectively, to detect the positions of the maxilla 10 and mandible 20 in real time without the influence of blind spots, thereby accurately analyzing the movements of the maxilla 10 and mandible 20.

When the maxilla 10 and mandible 20 are occluded, the exact position and shape of the occlusal surface when they are occluded may be derived from the first sensor unit 120 attached to the maxilla 10 and the second sensor unit 140 attached to the mandible 20. The apparatus 100 for tracking jaw motion in this example is configured to automatically generate a crown from the real-time analysis results of the occlusal surface by the control unit 180 (FIG. 1), and a doctor may immediately check the generated crown through the display 160 (FIG. 1) or make a surgical plan.

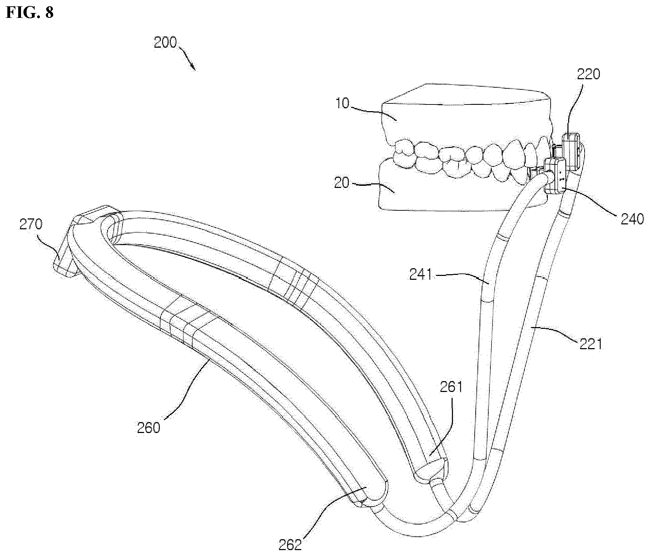

The drawings below FIG. 8 are exemplary diagrams illustrating a sensor assembly for tracking jaw motion related to the present invention. Here, an exemplary sensor assembly 200 that may be applied to the apparatus 100 for tracking jaw motion previously described in FIG. 1 is described.

As illustrated in FIG. 8, the sensor assembly 200 may include a first sensor unit 220 attached to the tooth 11 of the maxilla 10 and a second sensor unit 240 attached to the tooth 21 of the mandible 20. The first sensor unit 220 and the second sensor unit 240 may each include a magnetic sensor or an electromagnetic sensor for detecting the magnetic field or electromagnetic field of the base 110 (see FIG. 1). The first sensor unit 220 provides the detection signal for determining the movement or change in posture (roll, pitch, yaw, etc.) of the maxilla 10 independent of the second sensor unit 240 (based on a coordinate system defined by the X1, Y1, and Z1 axes) to the control unit 180 (see FIG. 1), and the second sensor unit 240 provides the detection signal for determining the movement or change in posture of the mandible 20 independent of the first sensor unit 220 (based on a coordinate system defined by the X2, Y2, and Z2 axes) to the control unit 180. The sensor assembly 200 may include a first cable 221 connected to the first sensor unit 220, a second cable 241 connected to the second sensor unit 240, a neckband 260 carrying the first cable 221 and the second cable 241 and an interface unit 270 arranged at a portion of the neckband 260.

The sensor assembly 200 of this example includes the neckband 260 that is a special structure to provide convenience of a patient while organizing the first cable 221 connected to the first sensor unit 220 attached to the tooth 11 of the maxilla 10 and the second cable 241 connected to the second sensor unit 240 attached to the tooth 21 of the mandible 20. The neckband 260 is formed in a 'C' shape that may be supported by being wrapped around a back of a neck of a patient, and has a front portion provided with end portions 261 and 262 to which the cables 221 and 241 are connected. The cables 221 and 241 are connected to one of these end portions 261 and 262, or as illustrated in FIG. 8, the first cable 221 may be configured to be connected to the first end portion 261 and the second cable 241 may be configured to be connected to the first end portion 261.

The neckband 260 may serve as a mechanism that may be hung around the neck, and make the cables 221 and 241 pass through the neckband 260 and connect the cables 221 and 241 to the interface unit 270. That is, in the present invention, the neckband 260 is configured to 'carry' the cables 221 and 241. Here, the 'carry' is used as meaning passing or supporting the cables 221 and 241. To this end, the neckband 260 may be designed to have a passage hole structure that makes the cables 221 and 241 pass through inside the neckband 260 or a clip-like structure that fixes the cables 221 and 241. The cables 221 and 241 have a structure that is electrically/physically connected to the end portions 261 and 262 of the neckband 260, and may be configured to enter the inside of the neckband 260 in a longitudinal direction or to be connected in a plug-in manner to the neckband 260.

As the mechanism structure of the neckband 260, a form (in this case, form restored to an original form when force is removed) that may be elastically opened when a small amount of force is used or a material or structure that may maintain the form when formed into a specific form using force may be applied.

The interface unit 270 may be formed in a connector structure with a plurality of pins and a board to be electrically connected to a cable of an external processing device. In addition, the interface unit 270 may also have communication means for being wirelessly connected to the external processing device, if necessary.

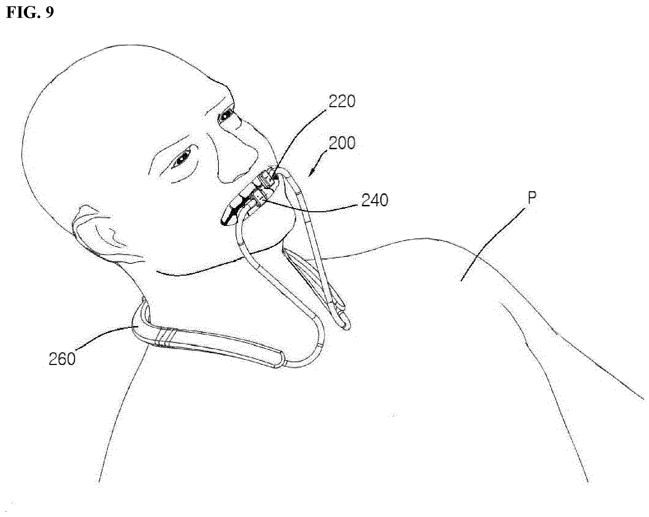

FIG. 9 illustrates a state in which a patient P is wearing the sensor assembly 200 related to the present invention. As the method of setting the sensor assembly 200 to the patient P, a method of allowing the patient P to first wear the neckband 260 and then attaching the first sensor unit 220 and the second sensor unit 240 to the teeth 11 of the maxilla 10 and the teeth 21 of the mandible 20, respectively, may be used, or conversely, a method of first attaching the first sensor unit 220 and the second sensor unit 240 and then wearing the neckband 260 on the patient P may be used.

When the cables 221 and 241 are connected to each sensor unit 220 and 240, the cables 221 and 241 may be configured to be connected to each sensor unit 220 and 240 at the side to minimize inconveniences such as covering a patient's face. However, the connection portions of the cables 221 and 241 to the sensor units 220 and 240 may be configured to be changeable or adjustable depending on the treatment area or treatment plan.

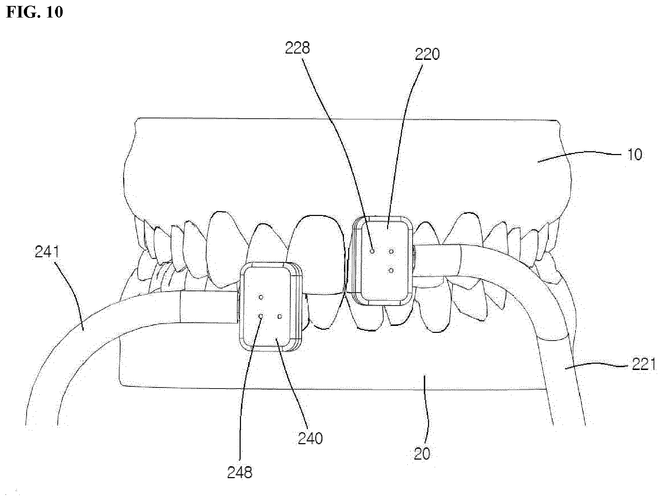

As illustrated in FIG. 10, similar to the description of FIGS. 3A and 3B, a plurality of beads 228 and 248 may be arranged in the first sensor unit 220 and the second sensor unit 240 to match the measurement data. However, for understanding, indication symbols for the beads 228 and 248 are marked only on one of the plurality of beads 228 included in the first sensor unit 220 and the plurality of beads 248 included in the sensor unit 240.

These beads 228 and 248 may be formed in the form of the magnetic metal bead and may have a triangular arrangement with a certain height difference in relation to each sensor board (not illustrated) inside. In this case, each housing or fixture constituting the first sensor unit 220 and the second sensor unit 240 may be made of non-magnetic material (e.g., titanium, synthetic resin, ceramic, etc.) to minimize magnetic interference. By doing this, when a patient moves the maxilla 10 and mandible 20 while the first sensor part 220 and the second sensor part 240 are attached to the maxilla 10 and mandible 20, respectively, the measured values of the first sensor part 220 and the second sensor part 240 are transmitted to the control unit 180, so the exact position, direction, and movement distance of the maxilla 10 and mandible 20 are derived in real time.

A method in which when the maxilla 10 and mandible 20 are occluded with each other, the first sensor unit 220 and the second sensor unit 240 has a separation distance to the extent that the teeth 11 of the maxilla 10 and the teeth 21 of the mandible 20 do not interfere with each other due to the naturally overlapping width, or as illustrated in FIGS. 4A and 4B, a method of arranging teeth to be fixed so that the teeth are staggered when viewed from above and below may be used.

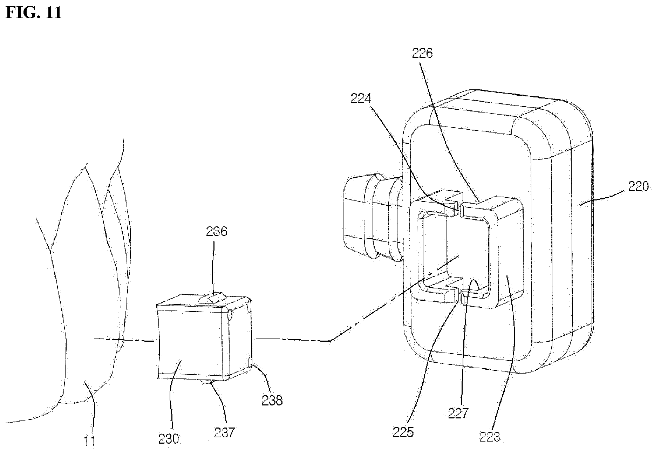

FIG. 11 illustrates that a fixing part 230 is used to fix the first sensor unit 220 to the tooth 11. The fixing part 230 is first fixed to a surface of the tooth 11 using adhesive means (e.g., photopolymerizable resin, etc.), and then the first sensor unit 220 is inserted into the fixing part 230 and fixed. The fixing method of the first sensor unit 220 may be applied to the fixing of the second sensor unit 240. That is, in order to fix the second sensor unit 240, the same fixing part (not illustrated) as the fixing part 230 of the first sensor unit 220 is provided. This will be replaced with a description of the method of fixing the first sensor unit 220.

In order to fix the first sensor unit 220 to the fixing part 230, a fastening shape part 223 to the fixing part 230 is formed on a rear side of the first sensor unit 220. The fastening shape part 223 may be formed in the form of a squared groove into which the fixing part 230, which is approximately cubic, is inserted. In order for the fastening shape part 223 to be firmly fixed to the fixing part 230, the fastening shape part 223 has cut grooves 224 and 225 formed on upper and lower sides thereof, respectively, and locking grooves 226 and 227 may be formed on the inside of the fixing part 230 for locking a locking protrusions 236 and 237 provided on the upper and lower surfaces of the fixing part 230, respectively.

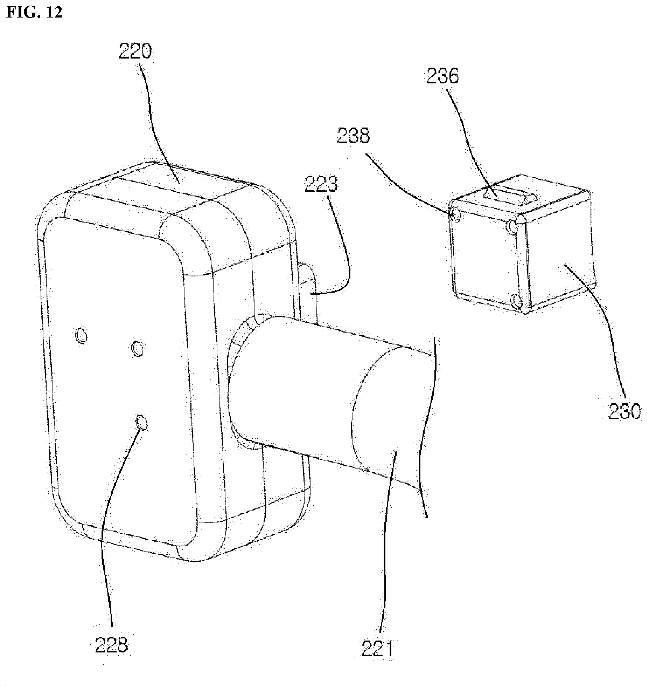

As illustrated in FIG. 12, a plurality of first beads 222 described above are arranged on a surface of the first sensor part 220. Separately from this, a plurality of beads 238 having the same arrangement as the first bead 222 are also arranged in the fixing part 230. The plurality of beads 238 are formed in the form of the magnetic metal bead so that they may be captured by the computed tomography device. The control unit 180 (FIG. 1) controls to generate the reference position from the plurality of beads 238 of the first fixing part 230 captured by the imaging device 170. In this case, when the first sensor unit 220 is inserted into the fixing part 230, the plurality of beads 238 of the fixing part 230 are arranged so that positions of the plurality of beads 238 of the fixing part 230 on the front view overlap with the plurality of first beads 228 of the first sensor unit 220. As described above, although not illustrated in the drawing, the plurality of beads (not illustrated) are also provided in the fixing part (not illustrated) to which the second sensor unit 240 is fixed. This is replaced with the description of the plurality of beads 238 of the fixing part 230 to which the first sensor unit 220 is fixed.

In this way, the first sensor unit 220 and the second sensor unit 240, which detect the magnetic field or electromagnetic field of the base 110, are attached to the tooth 11 of the maxilla 10 and the tooth 21 of the mandible 20, respectively, to detect the positions of the maxilla 10 and mandible 20 in real time without the influence of blind spots, thereby accurately tracking and analyzing the movements of the maxilla 10 and mandible 20. In this case, the cables 221 and 241 connecting the sensor units 220 and 240 are carried by the neckband 260 hung around the neck of the patient, and are connected to the external processing device through the interface unit 270, so the cables 221 and 241 of the sensor units 220 and 240 may be neatly organized and discomfort to patients or doctors may not be caused.

According to the apparatus and method for tracking jaw motion of the present invention, by attaching the sensor unit, which detects the magnetic or electromagnetic field of the base, to the maxillary teeth and the mandibular teeth, respectively, to detect the positions of the maxilla and mandible in real time without the influence of blind spots, it is possible to accurately analyze the movement of the mandible with respect to the maxilla. Unlike the optical method, since the miniaturized sensor unit that detects the magnetic field does not require the separate marker, and the apparatus is simplified, it is possible to minimize the impact on oral activity and make the joint movement of the patient free. In addition, since the required preparation process other than attaching the small sensor unit to teeth is small, there is an advantage in that convenience is improved and there is no need to configure a complicated system for each patient.

As an example related to the present invention, since the sensor part included in the sensor unit is fastened to the fixing part, which is first attached to teeth, in a detachable type (slide or dovetail method, snap fit method, etc.), it is easy to miniaturize the fixing part into the form of the bracket and design the sensor part in the form that can be fastened while minimizing the impact or interference on oral activities of the patient.

As an example related to the present invention, by using the first magnetic or electromagnetic sensor unit attached to the maxilla and the second magnetic or electromagnetic sensor unit attached to the mandible, it is possible to derive the exact position and shape of the occlusal surface when the maxilla and mandible are occluded, and by generating the crown from the results, it is possible to maximize the usability of the apparatus.

In addition, according to the sensor assembly of the present invention, since the cable connecting the sensor unit has the structure that is carried by the neckband hung around the neck of the patient and is connected to an external processing device through the interface unit, it is possible to neatly organize the cable of the sensor unit and improve the convenience by allowing the patient to move his or her body without causing discomfort to the patient or doctor.

The apparatus and method for tracking jaw motion and the sensor assembly therefor described above are not limited to the configuration and method of the described embodiments. All or some of the respective exemplary embodiments may be selectively combined with each other so that the above-mentioned exemplary embodiments may be variously modified to substitutable equivalents.

## Claims

1. An apparatus for tracking jaw motion, comprising:
a base that is configured to generate an electromagnetic field;
a first sensor unit that is attached to a maxillary tooth of a patient and includes a first sensor detecting the electromagnetic field generated from the base;
a second sensor unit that is attached to a mandibular tooth of the patient and includes a second sensor detecting the electromagnetic field generated from the base; and
a control unit that is configured to receive a detection signal from the first sensor and a detection signal from the second sensor, respectively, and calculate and provide a result of tracking a motion of a mandible with respect to a maxilla.

2. The apparatus of claim 1, wherein the first sensor unit includes:
a first fixing part that is attached to the maxillary tooth and preferably includes a first adhesive surface for attachment to the maxillary tooth by an orthodontic adhesive; and
a first sensor part that is detachably configured to the first fixing part and preferably includes a first slide groove that is configured to be inserted into the first fixing part in a slide manner, and
the second sensor unit includes:
a second fixing part that is attached to the mandibular tooth and preferably includes a second adhesive surface for attachment to the mandibular tooth by the orthodontic adhesive; and
a second sensor part that is detachably configured to the second fixing part and preferably includes a second slide groove that is configured to be inserted into the second fixing part in the slide manner.

3. The apparatus of claim 2, wherein a first protruding part protruding toward a first slide groove is formed on the first fixing part preferably having a frist through hole formed at its rear part, and/or
a second protruding part protruding toward a second slide groove is formed on the second fixing part preferably having a second through hole formed at its rear part .

4. The apparatus of claim 3, wherein a pair of first openings communicating with the first through hole is formed on both sides of the first protrusion, and/or
a pair of second openings communicating with the second through hole is formed on both sides of the second protrusion.

5. The apparatus of claim 3 or 4, wherein a first locking groove in which the first protrusion is caught in a snap fit shape is formed in the first slide groove, and/or
a second locking groove in which the second protrusion is caught in a snap fit shape is formed in the second slide groove.

6. The apparatus of one of the claims 2 to 5, wherein a plurality of first magnetic beads are arranged to be spaced apart from each other in the first sensor part, and/or
a plurality of second magnetic beads are arranged to be spaced apart from each other in the second sensor part.

7. The apparatus of claim 6, wherein a plurality of third magnetic beads are arranged to be spaced apart from each other in the first fixing part, and
a plurality of fourth magnetic beads are arranged to be spaced apart from each other in the second fixing part,
wherein the plurality of third beads and the plurality of fourth beads are preferably configured to be captured by an imaging device and the control unit controls to generate a reference position from the plurality of third beads and the plurality of fourth beads captured by the imaging device.

8. The apparatus of claim 7, wherein when the first sensor part is inserted into the first fixing part, the plurality of third beads are formed so that positions of the plurality of third beads on a front view overlap with the plurality of first beads, and/or
when the second sensor part is inserted into the second fixing part, the plurality of fourth beads are formed so that positions of the plurality of fourth beads on the front view overlap with the plurality of second beads.

9. The apparatus of one of the claims 2 to 8, wherein each body constituting the first fixing part and the second fixing part is made of a non-magnetic material, and/or
each housing supporting components included in the first sensor part and the second sensor part is made of the non-magnetic material.

10. A method for tracking jaw motion, comprising:
arranging a base that is configured to generate an electromagnetic field;
attaching a first sensor detecting the electromagnetic field generated from the base to a maxillary tooth of a patient;
attaching a second sensor detecting the electromagnetic field generated from the base to a mandibular tooth of the patient; and
receiving a detection signal from the first sensor and a detection signal from the second sensor, respectively, and calculating and providing a result of tracking a motion of a mandible with respect to a maxilla.

11. The method of claim 10, further comprising:
automatically generating a crown from the result of deriving a position and shape of an occlusal surface when the maxilla and the mandible are occluded.

12. A sensor assembly for tracking jaw motion, comprising:
a first sensor unit that is attached to a maxillary tooth of a patient and configured to detect an electromagnetic field generated from a base;
a first cable that transmits a detection signal of the first sensor unit;
a second sensor unit that is attached to a mandibular tooth of the patient and configured to detect the electromagnetic field generated from the base;
a second cable that transmits a detection signal of the second sensor unit;
a neckband that is configured to be worn around a neck of the patient and carries the first cable and the second cable; and
an interface unit that is provided at a portion of the neckband and configured to allow the cables to be connected to an external processing device.

13. The sensor assembly of claim 12, wherein the neckband is formed in a 'C' shape that is supported by being wrapped around a back of the neck of the patient, and has a front portion provided with a first end portion to which the first cable is connected and a second end portion to which the second cable is connected.

14. The sensor assembly of claim 13, wherein the neckband is formed so that the first cable and the second cable follow the neckband from the first end portion and the second end portion provided at a front portion thereof, respectively, or are each arranged inside the neckband.

15. The sensor assembly of claim 12 or 13, wherein the first sensor unit and the second sensor unit are each configured to be detachably coupled to a fixing part attached to teeth of the patient.
